# EUROPEAN PATENT APPLICATION

(11) **EP 4 023 753 A1**
(43) Date of publication of application: **06.07.2022**
(21) Application number: 20854378.5
(22) Date of filing: 19.08.2020
(51) Int. Cl.: C12N 15/10, G01N 1/00

(54) **RNA-FREE ANIMAL SERUM**

(30) Priority: 19.08.2019 MX 2019009864
(71) Applicant: Instituto Nacional de Medicina Genómica, Ciuded de México, 14610 (MX)
(72) Inventor: FLORES JASSO, Carlos Fabián, Ciudad de México, 01860 (MX); AVENDAÑO VÁZQUEZ, Selma Eréndira, Ciudad de México, 01860 (MX); FLORES TORRES, Mariana, Ciudad de México, 03920 (MX); CHAVIRA DESALES, Diana, Ciudad de México, 03300 (MX)
(74) Representative: Padial Martinez, Ana Belen
(86) International application number: PCT/MX2020/050027
(87) International publication number: WO 2021/034181

(57) **Abstract**

The present invention relates to RNA-free mammal serums which can be useful for cell culture or for producing pharmaco-biological products, due to the fact that they maintain their supplement features. Another embodiment of the invention refers to the method for removing RNA from mammal serum through the application of sequential serum heating, alkalisation and neutralisation steps.

## Description

### TECHNICAL FIELD

The present invention is inserted within the field of cell biology and specifically, within the field of supplements for cell culture. In general, the present invention relates to a serum, preferably fetal bovine serum, reduced in ribonucleic acid (RNA) or with a minimal content of RNA that is below the detection limit and to a method useful to remove RNA from serum. This RNA-reduced serum is useful for analyzing the expression of RNA from cell cultures, without interfering in said analyzes by the RNA normally present in the serum of animals.

### BACKGROUND

Cell culture is one of the main tools for basic and biomedical *in vitro* research. It consists of keeping cells from very different types of animals, from insects to mammals, under controlled conditions of temperature, humidity and percentage of carbon dioxide. To keep cells alive and in constant division, cell cultures are kept in liquid media containing the appropriate amount of salts for cells to carry out their most essential metabolic functions (Swain P. Basic Techniques and Limitations in Establishing Cell Culture: a Mini Review. Adv Anim Vet Sci (2014) doi: 10.14737/journal.aavs/2014/2.4s.1.10 and Arora M. Cell Culture Media: A Review. Mater Methods (2013) doi: 10.13070/mm.en.3.175).

An important component for *in vitro* cell culture is serum, as it contains a high content of growth factors, lipids, proteins and other necessary factors that allow cells to keep in constant division. In this way, serum is added as a nutritional supplement to synthetic liquid media that maintain cell cultures. The serum used for this purpose can be of human, equine or bovine origin, being the fetal bovine serum (FBS) the most widely used worldwide (Arora M. Cell Culture Media: A Review. Mater Methods (2013) doi: 10.13070/mm.in.3.175.)

In addition to the aforementioned components, FBS has a high content of nucleic acids, the most abundant being RNA in its several biologically functional forms such as transfer RNA (tRNA), ribosomal RNA (rRNA), micro RNA (miRNA), RNAs associated with Piwi (pi-RNA), nucleolar RNA (snoRNAs), among others (Chen X, et al. Cell Res. 2008 Oct; 18 (10): 997-1006). Some of the various types of RNA are contained within extracellular vesicles (EVs) and others outside same (Keerthikumar S. et al. J Mol Biol. 2016 Feb 22; 428 (4): 688-692).

EVs are 50-400 nm diameter microstructures made up of a lipid bilayer and containing proteins, the various types of biologically functional RNA, and some small molecules. EVs are formed by subgroups thereof that differ from each other by diameter and molecular content that comprise them, with exosomes, 50-120 nm diameter EVs, being the most widely studied ones (Colombo M. et al. Annu Rev Cell Dev Biol. 2014; 30: 255-89). An important aspect of EVs is that they have been shown to be capable of transferring their content to recipient cells, both *in vitro* and *in vivo;* therefore, they have been proposed as an intercellular communication system that may be of great importance in metabolism, as well as in the development of diseases such as cancer and diabetes, among many others (Muralidharan-Chari V. et al. J Cell Sci. 2010 May 15; 123 (Pt 10): 1603-11 and Bobrie A. et al. Traffic. 2011 Dec; 12 (12): 1659-68).

Studies in cells in culture demonstrated that the EVs of the FBS are capable of transferring their RNA content to human and murine cell lines; highlighting the possible transfer of bovine RNA within cell cultures when using FBS. This finding disclosed that since the discovery of miRNAs to date, it is highly probable that the scientific literature reported on the detection and analysis of miRNAs expression is a mixture of bovine RNA with that of the species from which the cell line of the study at hand comes. The magnitude of the degree of interference, both in the detection of bovine RNA and in its possible function within cell cultures, is not known to date. The main reason for this is that more than 70% of cow miRNAs are identical to those of other mammals, including those of humans (Wei Z. et al. Sci Rep. 2016 Aug 9; 6: 31175).

In this sense, Wei et al. describe that FBS contains various types of RNA, both protein-coding RNA and regulatory RNA, including messenger RNA, microRNAs (miRNAs), ribosomal RNA, and small nuclear RNA, from which up to 70% can remain in the serum even after applying ultracentrifugation processes.

Wei et al. indicate that the RNA proper to FBS is isolated together with the RNA derived from cell cultures, which may cause interferences or misinterpretations in subsequent RNA analyzes, which is why it is desirable to have an RNA-free serum to carry out this type of studies (Tosar JP. J Extracell Vesicles. 2017 Jan 12; 6 (1): 1272832).

In order to reduce possible interferences or misinterpretations, due to the presence of endogenous EVs in the serum used for cell cultures, several methods have been developed to eliminate EVs from serum.

In this sense, US patent 9,005,888 describes methods for the isolation of EVs produced by animal cells and methods to produce EV content-reduced plasma or serum, indicating that some of the miRNAs originally present are undetectable by quantitative PCR (qPCR). after applying the method. However, the method described in US patent 9,005,888 has several disadvantages, including the use of a precipitation solution containing polyethylene glycol (PEG) of which residues remain in the treated serum and which in turn can be considered as a pollutant element.

It has been described, for example, that PEG can induce the formation of heterokaryotes, that is, cell fusion to obtain multinucleated cells (Davidson RL, Gerald PS. Somatic Cell Genet. 1976 Mar; 2(2):165-76.), so that the PEG remaining in the FBS could alter the biological function of cells, generating altered results in molecular biology experiments.

In addition to this, it cannot be ruled out that the remaining PEG in serum affects the extraction of RNA by conventional methods, for example Trizol, and that the effect of the little or no detection of RNA described in US patent 9,005,888 is due to an artifact caused by PEG and not to RNA removal as such.

It is noteworthy that most of the serum RNA is not contained within EVs but outside thereof, as described by Wei et al.; which greatly limits the certainty that there is no RNA pollution in cell cultures when using PEG-treated sera. Likewise, despite the fact that with the PEG precipitation method it is also possible to precipitate proteins in high concentration from the serum, it is unknown if it is useful to completely remove circulating RNA associated-protein complexes, exogenous to the EVs content (Tosar et al.) .

It is also important to point out that the method described in US patent 9,005,888 requires steps and serum manipulations that jeopardize the sterility conditions thereof, which could force the inclusion of an additional step to return the serum to its sterile condition once it has been treated with PEG; this, in order to use the FBS in sterile cell cultures. This would increase the time and expense generated to produce the serum without EVs. Another important aspect is that the PEG method does not completely remove the EVs present in the serum since one of the embodiments described defines the EVs-reduced serum with a concentration of no more than 104 vesicles per milliliter.

For their part, Kornilov et al. (Kornilov R. et al. J Extracell Vesicles. 2018 Jan 21; 7(1):1422674) describe a method to remove EVs from FBS by ultrafiltration. Despite offering a methodologically simpler laboratory-level alternative to eliminate EVs, this methodology still requires centrifugation and the use of expensive materials (such as ultrafiltration materials and equipment), which reduces the practicality of the methodology and the application thereof at an industrial level. In addition, this method reduces the amount of EVs, but the RNA that is outside of same is not removed, so the serum treated in this way still contains significant amounts of RNA of FBS.

In addition to the aforementioned methods for treating FBS and reducing EVs content as well as the RNA amount thereof, there are commercial alternatives for FBS substitutes for cell culture. Such serum substitutes are synthetic formulations that contain part of the natural components of serum, in such a way that some cell lines are capable of growing in optimal conditions under controlled culture conditions (Barnes D, Sato G. Anal Biochem. 1980 Mar 1; 102 (2) 255-70). However, synthetic serum substitutes tend to be radically more expensive than FBS and are generally designed to grow specific types of cells, so, in general, they only represent an effective FBS replacement alternative for growing cell lines and/or specific culture conditions, for example, for stem cells, Ohnuma K. et al. J Neurosci Methods. 2006 Mar 15; 151 (2):250-61).

Derived from the need to study the biological effect that exosomes in cell culture pose and prevent serum miRNAs from contaminating the cultures, and therefore, hinder the conclusions of the experiments, there are already miRNAs reduced-FBS products in the market (Paszkiet B. et al. Development of an improved process for the depletion of exosomes from fetal bovine serum. Thermo Fisher Scientific Inc. 2016), however, these contain significant amounts of other types of RNA whose effects on culture have not been studied.

Ideally, to avoid RNA contamination within cell cultures, the serum used to supplement the media should be free or substantially reduced in RNA and free or substantially reduced in EVs, but it is also desirable that it does not contain any additional chemical compounds, such as PEG, for example. However, this serum should retain the other serum components that are essential for cell culture.

Based on the current prior art, the need for an animal serum, mainly FBS, that is RNA-free or with a minimum content of RNA that allows carrying out research in cell cultures without risk of contamination or interference with the RNA of the serum, is evident. Likewise, the need for a method to remove RNA from animal origin serum used for cell culture by means of simple and low-cost techniques that do not put serum sterility at risk and that are industrially scalable without radically increasing the cost of the serum, is obvious.

### SUMMARY OF THE INVENTION

The present invention relates to an RNA-free or substantially RNA-free animal serum. The serum is in turn free or substantially free of extracellular vesicles (EVs), but it maintains the other components of the serum that are essential for cell culture, making it useful to carry out research based on cell cultures without risk of contamination by EVs or RNA of the serum.

Within the embodiments of the invention, different types of animal serum such as bovine, equine, human, mouse, rat and goat serum are included, in addition to the respective fetal serum variants.

The present application also refers to a method useful to remove or substantially reduce the amount of RNA present in an animal serum, mainly Fetal Bovine Serum (FBS), through the sequential application of heating, cooling, alkalinization and neutralization processes. This method breaks down the endogenous EVs that are present in the animal serum and in turn denatures and degrades the free RNA in the serum, which allows the RNA present in said serum to be removed or substantially reduced. The disintegration of EVs nullifies the natural functionality thereof.

### BRIEF DESCRIPTION OF THE DRAWINGS

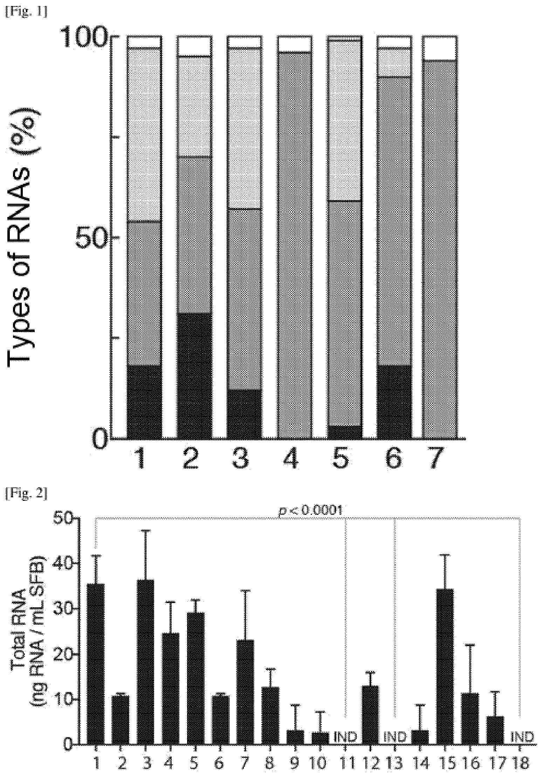

Figure 1 shows a bar graph that presents a summary of the ratios of the classes and abundance of RNA in different commercial FBS products. Each bar shows a different FBS packaging. 1) Regular FBS A; 2) regular FBS B; 3) characterized FBS; 4) Qualified FBS; 5) certified FBS; 6) Triple filtered FBS; 7) exosomes reduced-FBS. It is noted that the contents of the different classes and the amounts of RNA differ with respect to the packaging and, although some do not present miRNAs, all present RNA. The black bars represent miRNAs; the dark gray bars represent non-annotated RNAs (sequences that are found in the Bos taurus genome but that could not be associated with a type of RNA with specific function); light gray bars represent tRNAs; and the white bars represent other RNAs not found in the Bos taurus genome, whose origin may be non-bovine.
Figure 2 shows a graph showing the concentration of RNA recovered after combining the different processes applied to FBS samples to achieve RNA removal. The processes used and their order are described in Table 1. RNA was removed under three different conditions (bars 11, 13 and 18). IND indicates that the RNA concentration was undetectable. The combination of inactivation by heating, cooling, alkalinization and neutralization in that specific order, is one of the combinations that produces the removal or substantial decrease of RNA in FBS. The other combinations that reduce the amount of RNA involve an additional ultracentrifugation step.

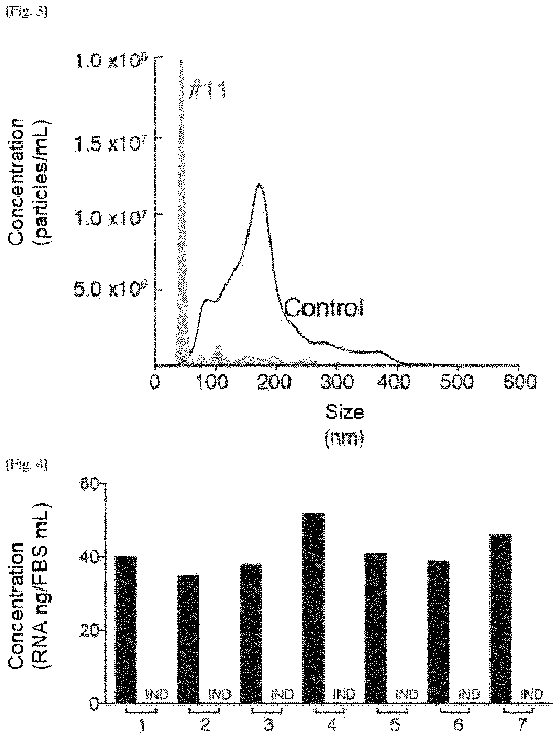

Figure 3 shows a histogram that describes the amount and size of the particles contained in the FBS. The histogram in gray color corresponds to the size and amount of the particles present in a FBS sample treated with the method of the invention (combination # 11 according to Table 1), and the black contour histogram corresponds to the control sample without no treatment. Particles of the representative size of EVs (50 to 400 nm in diameter), including exosomes, were reduced in size compared to untreated serum (control), with the vast majority having a diameter of about 30 nm after applying heating, cooling, alkalinization and neutralization.
Figure 4 shows a bar graph showing the RNA concentrations of several commercial FBS products before and after the application of the method of the invention. 1) Regular FBS A; 2) regular FBS B; 3) characterized FBS; 4) Qualified FBS; 5) certified FBS; 6) Triple filtered FBS; 7) exosome reduced-FBS. It is noted that regardless of the commercial product and its initial RNA concentration, the RNA concentration is undetectable (IND) after applying the RNA removal method described in the present application.

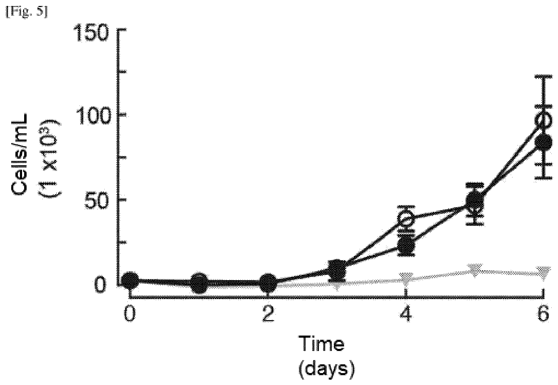

Figure 5 is a graph showing the cell proliferation of a cell culture wherein the RNA-free or substantially RNA-free FBS described in the present invention is used as a complement. HEK 293 cells were grown under different supplement conditions: white circles represent untreated FBS (control), black circles represent RNA-free or substantially RNA-free FBS of the invention (obtained with combination # 11 of Table 1) and the gray triangles represent a synthetic serum substitute. Cells incubated with RNA-free or substantially RNA-free FBS show a proliferation similar to that of cells cultured with untreated FBS (control), and higher than that of the synthetic substitute, showng that the RNA-free or substantially RNA-free FBS obtained after applying the method of the invention maintains the properties needed to effectively promote cell proliferation.

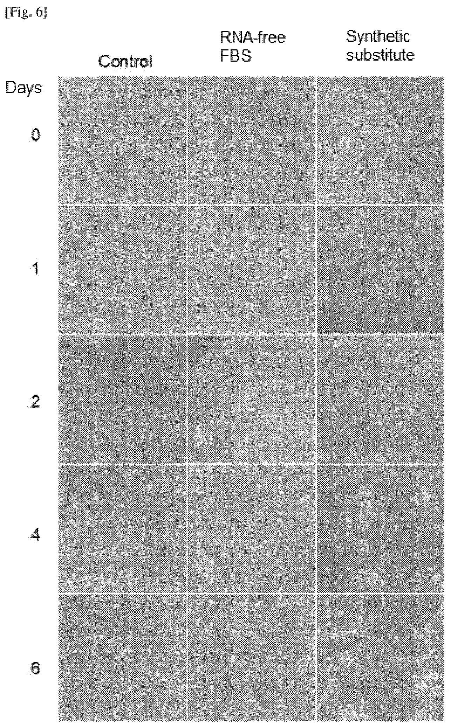

Figure 6 shows the morphology of cultured cells using different supplement conditions. Untreated FBS (control), RNA-free or substantially RNA-free FBS described in the present invention (obtained by combination # 11 described in Table 1) and synthetic serum substitute were used. It is noted that the RNA-free or substantially RNA-free FBS of the invention does not have a negative influence on the morphology of the cells with respect to the control, nor with respect to the synthetic serum, demonstrating that it maintains the properties needed for cell proliferation..

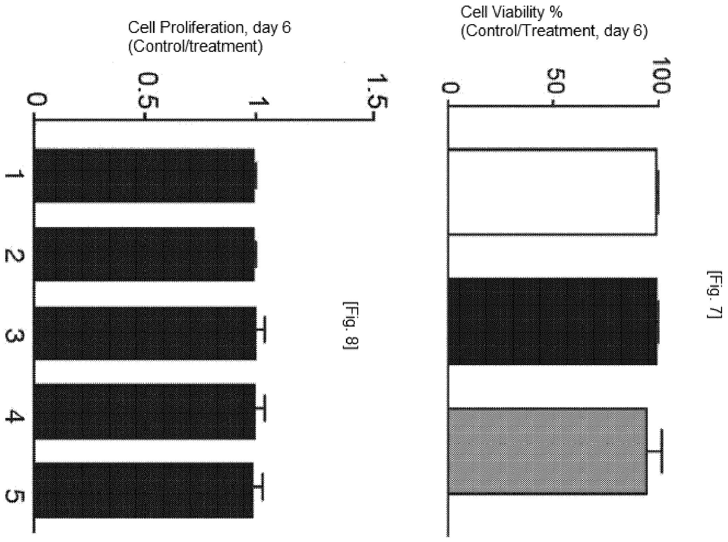

Figure 7 shows a bar graph where a comparison of the cell viability of cultures under different supplement conditions is presented. FBS was used untreated (control), white bar; the RNA-free or substantially RNA-free FBS of the invention, black bar, and a synthetic serum substitute, gray bar. It is noted that there are no significant differences in the accumulated viability on day 6 between the cells supplemented with the control FBS and the RNA-free FBS and the synthetic substitute, confirming that the RNA-free serum maintains its supplement properties after the application of the method of the invention.
Figure 8 shows the ratio between the proliferation of cell lines supplemented with RNA-free FBS and with untreated serum as control: 1) HEK 293, 2) HeLa, 3) CHO, 4) MCF7 and 5) MEF cells supplemented with untreated FBS (control) and with RNA-free or substantially RNA-free FBS were incubated, obtaining the proliferation ratio under both supplement conditions. None of the cell lines incubated with RNA-free FBS showed differences in cumulative proliferation on day 6, with respect to untreated FBS supplementation.

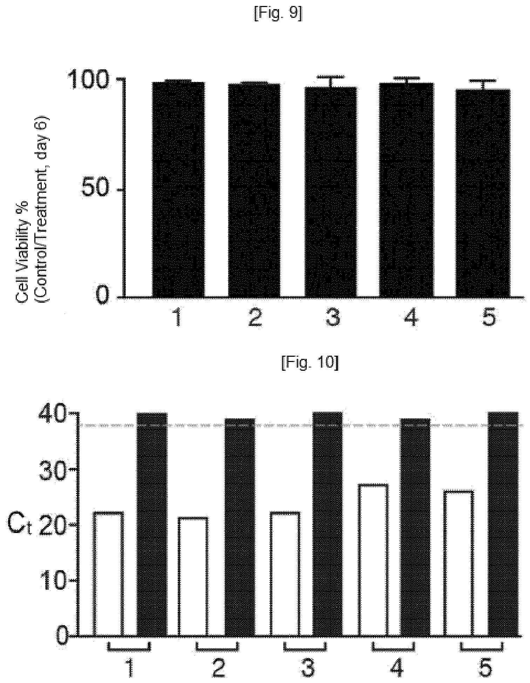

Figure 9 shows the ratio between the cell viability of lines supplemented with RNA-free FBS and with untreated serum (control). 1) HEK 293, 2) HeLa, 3) CHO, 4) MCF7 and 5) MEF cells supplemented with untreated FBS and with RNA-free or substantially RNA-free FBS were incubated, obtaining the viability ratio under both supplement conditions. None of the cell lines incubated with RNA-free FBS show differences in cumulative viability at day 6 compared to their respective FBS control.
Figure 10 shows a graph of the amount of several miRNAs in an untreated serum (control) and in the same serum after being treated with the method of the invention. 1) Bta-miR-143; 2) Bta-miR-181a; 3) Bta-miR-192; 4) Bta-miR-380-3p and 5) Hsa-miR-25-3p miRNAs were detected by quantitative reverse transcription PCR (qRT-PCR). The white bars represent the untreated FBS and the black bars the FBS treated with the method of the invention. In all cases, the method of the invention decreased the amount of miRNAs to levels below the detection limit (about 38 Ct), showing the effectiveness of the method to remove miRNAs from FBS.

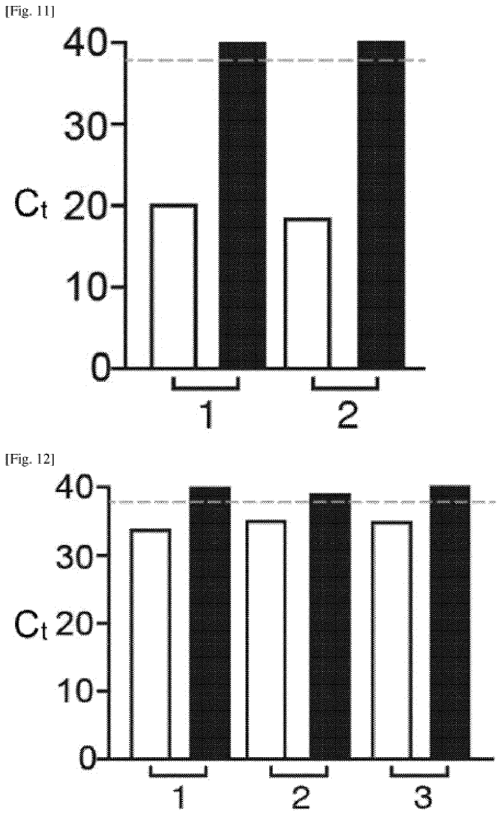

Figure 11 shows a graph of the amount of other classes of RNA in an untreated serum (control) and in the same serum after being treated with the method of the invention. 1) U47 and 2) unannotated RNA # 49627 RNAs were detected by qRT-PCR. White bars represent the untreated FBS (control) and the black bars represent the FBS after treatment with the method of the invention, making it clear that the method is effective in removing several kinds of RNA.
Figure 12 shows a graph of the amount of several miRNAs in an untreated serum (control) and in the same serum after being treated with the method of the invention. 1) hsa-miR-486; 2) hsa-miR-423-5p; and 3) hsa-miR-10b miRNAs were detected by qRT-PCR. The white bars represent the Untreated FBS (control) and the black bars represent the FBS after treatment with the method of the invention. As can be seen, these miRNAs are of low amount in the serum sample used, however, the method of the invention is capable of removing same to undetectable levels.

### DISCLOSURE OF THE INVENTION

The culture of cell lines and primary tissues is one of the main tools for the study of biomedical research. Typically, cell culture is carried out by incubating cells in liquid media containing supplements that allow the cells to be kept alive or in constant proliferation. Fetal bovine serum (FBS) serves as a supplement to the culture medium because it is composed of a complex mixture of proteins, nucleic acids, hormones, growth factors, lipids, and other small molecules such as vitamins and minerals that are important for cellular growth.

FBS contains a mixture of different classes of RNA, including miRNAs, which are highly conserved, so there are miRNAs that are exactly the same in sequence and size between evolutionarily distant species. Due to their high degree of conservation and specificity, miRNAs are considered important regulators of gene expression (Bartel D. P. Metazoan MicroRNAs. Cell (2018) 173: 20-51).

It was recently reported that RNA present in FBS can interfere with the detection of intracellular miRNAs when serum is used as a supplement in cell cultures (Wei Z. et al. Sci Rep. 2016 Aug 9; 6: 31175). This discovery has great significance in molecular biology since the vast majority of studies where RNA gene expression is characterized in cell cultures uses FBS as a complement and the results obtained could be altered by a mixed effect of the miRNAs typical of the cultured cells and those from FBS. This situation is aggravated when considering that in a significant number of miRNAs it is not possible to distinguish the species from which they come. Nor does it rule out the possibility that miRNAs or other RNAs present in the sera used as a supplement may exert a biological function on cells in culture, either by altering the expression of genes, or their physiological state, and therefore, the results of the experiments carried out.

One of the possible sources of the bovine miRNAs transfer to cell cultures are the extracellular vesicles (EVs) contained in the FBS, however, the pollution with bovine RNA also comes from the extra-vesicular medium since the amount miRNAs and other types of RNA are present in equivalent ratios both within the EVs and outside same, so that the removal of the EVs from the serum is not sufficient to completely remove the contaminating RNA.

Finally, it is important to note that the RNA contained in the FBS is very stable, either inside or outside the VEs, so it should be protected by RNA-binding proteins, since naturally, the FBS contains a large amount of RNAses that would degrade RNA if it were not protected effectively (Chen X, et al. Cell Res. 2008 Oct; 18 (10): 997-1006).

In the prior art, several methods are described to reduce or remove VEs from FBS, which consist of ultrafiltration, ultracentrifugation and precipitation with the use of chemical agents, however, these methods are focused on the elimination of EVs and the biological material contained within these EVs, but they are not capable of reducing or removing the RNA that is found in free form in serum, and that can represent up to 60% of the RNA contained in mammalian serum, so none of these methods or formulations of FBS is free or substantially free of RNA. A possible alternative is the use of synthetic supplements, but these are often much more expensive than the use of FBS and are not effective for the growth of all cell types.

This is why it is desirable to have RNA-free or substantially RNA-reduced mammalian serum, which allows cell culture to be carried out *in vitro,* without causing contamination or possible alteration of the results of molecular biology experiments and which at the same time maintains its characteristics and functionality as a supplement. In this sense, it is also desirable to have a method that allows the production of an RNA-free or substantially RNA-reduced serum without altering the function thereof as a supplement and without leaving residues of chemical agents that potentially interfere with cell culture or molecular biology experiments in which they are used.

The present invention overcomes the shortcomings of the prior art by providing RNA-free or substantially RNA-reduced serum, as well as an efficient method for removing RNA contained in mammalian serum, particularly FBS. This method is effective in eliminating the RNA present in mammalian serum, whether it is contained within the EVs or outside same, thus producing sera from different species of mammals that are free or substantially reduced in RNA.

Another technical advantage of the method described is that it is a simple method that does not require centrifugation or ultracentrifugation, or the use of filters, which reduces application costs and industrial scaling. Furthermore, the method can be carried out without the need to transfer the serum into different containers, which facilitates maintaining the sterile conditions required for its use. The serum produced from the method described in the present application is free or substantially reduced in EVs and in total RNA, reaching a higher rate of RNA removal with respect to other sera described in the prior art.

The present invention is based on the unexpected fact that the application of heating, cooling, alkalinization and neutralization processes, in that specific order, on mammalian serum, results in a substantial elimination or decrease of the RNA that is naturally present in serum. Despite the fact that these processes are in common use and the effect of each of them on the different biomolecules present in serum is known, a method that combines the four processes with this particular order of execution and having this effectiveness to remove RNA, had not been described so far. The specific order of application of these processes, as described in the present invention, increases the removal efficiency of RNA present in mammalian serum.

Since the present invention is based on the application of the heating, cooling, alkalinization and neutralization processes in that specific order, which is counter-intuitive to the current prior art, it is not expected for an ordinary skilled technician to reach similar conclusions. Applying these processes separately or in a different sequence in serum does not produce the same results, so the present method is not obvious from the prior art.

RNA-free or substantially RNA-reduced serum can be used as a supplement for any type *of in vitro* cell culture (for example, HeLa, HEK 293, CHO, MCF7, MEF, among others) and for any molecular biology experiment seeking to investigate the metabolic or physiological status of the cell under study. Also, RNA-free or substantially RNA-reduced serum is useful as a complement in all those studies associated with the analysis of gene expression and more particularly with the analysis of expression of several classes of RNA, for example miRNAs, tRNAs, IncRNAs, mRNAs, snRNAs and piRNAs, among others. Similarly, RNA-free or substantially RNA-reduced serum can be used as a supplement for the culture of cells useful in the production of therapeutic agents such as hormones, recombinant proteins, antibodies, clotting factors and vaccines, to name a few (Ashish Verma, Anchal Singh Academic Press, Nov 4, 2013).

As used in the specification of the present application, the terms "RNA-free serum" and "substantially RNA-reduced serum" refer to an animal serum, preferably of mammalian origin, that has been subjected to a process of removal of RNA and whose endogenous RNA content is at undetectable levels by highly sensitive conventional methods useful for measuring nucleic acid concentration in aqueous samples, for example, spectrophotometry and spectro-fluorometry, or by detecting specific RNA sequences by methods that detect their presence, and therefore, their relative amount, and that the little or no detection of RNA sequences is typically interpreted as "absent" or "undetectable" sequence, as is the case of qRT-PCR, wherein the amplification cycles (Ct) are indicative of the amount of the sequence of interest, considering that the amount signals with Ct greater than 38 cycles are indicative of the a absence, or the null detection of the sequence sought. In specific sequence detection techniques such as next generation sequencing (RNA-seq, small RNA-seq), it is conventionally assumed that the number of reads is indicative of the amount of the sequence of interest, such that a sequence with 0 readings indicates that RNA is absent or below the detection limit of the technique.

For the development of the present invention, the composition and relative amount of the different types of RNA present in the serum were analyzed in the first instance (Figure 1). For this, samples of different commercial FBS products: 1) regular FBS A; 2) regular FBS B; 3) characterized FBS; 4) Qualified FBS; 5) certified FBS; 6) Triple filtered FBS and 7) exosome reduced-FBS were analyzed by the RNAseq method (Illumina). The results of the bioinformatic analysis shows that FBS contains different classes of RNA in different amounts. Among the most abundant RNA classes we find miRNAs, tRNAs, and unannoted RNAs (whose sequence is located in the Bos taurus genome, but which could not be associated with a specific type of RNA). It is important to note that the ratios of the different types of RNA vary between the different commercial products, and some of them show a low ratio of miRNAs compared to the others, but all show some type of RNA.

To determine the most suitable conditions for the process of removal of the RNA present in the serum, different processes and combinations thereof were tested, including: heating, cooling, alkalinization, addition of enzymes (ribonucleases) and ultracentrifugation. The combinations of the different processes examined are described in Table 1. After applying the processes, the RNA was extracted with the TRIzol (Thermo) reagent and quantified with the RNA extracted by means of Qubit-type spectro-fluorometry (Thermo).

**Table 1**

| Combination | Treatment | | | | | | |
|---|---|---|---|---|---|---|---|
| | Untreated (control) | Heating | Cooling | Alkalnization | Neutralization | Enzymatic Degradation | Ultracentrifugation |
| 1 | X | | | | | | |
| 2 | | X | X | | | | |
| 3 | | | | X | X | | |
| 4 | | | | | | X | |
| 5 | | | | | | | X |
| 6 | | 1st | 2nd | 3rd | | | |
| 7 | | 2nd | 3rd | 1st | | | |
| 8 | | | | 1st | 2nd | | |
| 9 | | 1st | 2nd | | | 3rd | |
| 10 | | 2nd | 3rd | | | 1st | |
| 11 | | 1st | 2nd | 3rd | 4th | | |
| 12 | | 2nd | 3rd | 1st | 4th | | |
| 13 | | 1st | 2nd | | | | 3rd |
| 14 | | 2nd | 3th | | | | 4th |
| 15 | | | | 2nd | | | 1st |
| 16 | | | | | | 2nd | 1st |
| 17 | | | | 2nd | 3rd | | 1st |
| 18 | | 2nd | 3rd | 4th | 5th | | 1st |
| *RNase A [100µg/mL] (Qiagen) | | | | | | | |
| **Ultracentrifugation at 100,000 xg | | | | | | | |

Figure 2 shows the efficiency of RNA removal by different processes and their combinations in FBS samples. Only three specific combinations of the different processes lead to RNA removal to undetectable levels (IND), below the detection limit of 250 pg/µL by spectro-fluorometry. One of the combinations (Figure 2, bar # 11), consists of heating and cooling, followed by alkalinization and then neutralization. Another effective combination (Figure 2, bar # 13) consists of heating and cooling followed by ultracentrifugation. A third effective combination (Figure 2, bar # 18), is the combination of the ultracentrifugation steps, followed by heating and cooling, followed by alkalinization and then neutralization, in that sequential order.

Other processes and combinations show some degree of effectiveness in removing RNA from serum, but none of them are useful in removing it completely or below the detection limit. It is important to highlight that the removal of RNA from the serum is not only carried out by applying the four processes described: heating, cooling, alkalinization and neutralization in combination, but that the order in which these processes are applied is especially important, since the combination of the three processes, but in a different order (Figure 2, bar # 12), does not have the same effect on the removal of RNA from the serum; which is surprising and not obvious from the prior art. In conclusion, applying these processes separately or in a different sequence in the serum does not produce the removal of the RNA it contains.

Given that part of the RNA present in the serum is contained within EVs, a quantification of nanoparticles was carried out to verify the effect of the method of the invention on the EVs present in the FBS, by means of a nanoparticles tracking analysis (NTA) using a NanoSight (Malvern) instrument (Figure 3).

The size of the particles naturally contained in the FBS ranges between 50 and 400 nm in diameter, with two particle concentration peaks close to 100 and 180 nm (Figure 3, Control. Black contour histogram). After the application of the method described in the present application, there is a significant decrease in the size of the particles present in the FBS, having a maximum particle concentration peak close to 30 nm in diameter (Figure 3, # 11. Gray Histogram).

These results indicate that the application of the RNA removal method dissagregates the EVs present in the serum, including exosomes, since the peak seen around 30 nm has an amount (1.5 x 108 particles) that correlates with the sum of the quantity of particles in the two main peaks of the control sample, indicating a dissagregation of the natural status of the EVs, removing or significantly reducing the contents of EVs of the serum. The dissagregation of the EVs makes the removal of the total RNA present in the animal serum more efficient, probably releasing the RNA present within the EVs, for its subsequent degradation.

This result can be explained by the fact that the heating and cooling processes denature the EVs, releasing the RNA they contain, subsequently the treatment with a strong base produces the alkaline hydrolysis of the RNA and the addition of a strong acid promotes greater RNA degradation, resulting in an increase in the efficiency of RNA removal from serum. It is likely that the RNA remaining in the serum after this treatment is comprised by non-functional fragments, a product of the degradation of RNA molecules, so they would not have an effect on cells in culture.

To corroborate the efficiency of the method to remove RNA from serum, the RNA of seven different commercial FBS products was quantified before and after being treated with the method described in the present application. RNA was extracted with the TRIzol (Thermo) reagent, and the extracted RNA was quantified by Qubit-type spectro-fluorometry (Thermo).

The RNA removal method described in the present application is effective in all commercial FBS products that were tested: 1) regular FBS A; 2) regular FBS B; 3) characterized FBS; 4) Qualified FBS; 5) certified FBS; 6) triple filtered FBS and 7) exosome reduced-FBS. Figure 4 shows that despite having different content of RNA classes, as previously determined (Figure 1), the method is capable of removing the RNA of the different commercial products at levels below the detection limit, indicating that the method is effective regardless of the RNA concentration and of present in the serum and is therefore applicable to any type of animal serum.

Afterwards, it was evaluated whether the RNA-free or substantially RNA-reduced FBS described in the present application has alterations in terms of its supplementing capacity for cell culture after the application of the RNA removal method. For this, a growth curve of human cells (HEK 293 cells) supplemented with RNA-free serum was performed, and the proliferation, morphology and viability thereof for six days were determined, comparing them with those obtained from cells grown with medium supplemented with untreated serum and with a synthetic serum substitute, a synthetic formulation containing the necessary components for some types of cell lines to grow in culture, and which, due to its synthetic nature, does not contain RNA or EVs.

Figure 5 shows the comparison of cell proliferation in the three supplement conditions. HEK 293 cells were grown in culture medium under normal conditions, until reaching a 60% confluence. Afterwards, the culture medium was changed, incorporating media supplemented with any of the variants (control FBS, RNA-free FBS and synthetic serum substitute), quantifying the density of the cells (day 0-6). The result shows that there is no difference in the cell proliferation of the culture supplemented with the RNA-free serum (black circles), compared to the untreated FBS (control, open circles). The proliferation obtained using RNA-free serum is higher than that of cells grown in medium supplemented with the synthetic serum substitute (gray triangles).

Figure 6 shows the morphology of the cells cultured under the three supplement conditions. It is noted that the RNA-free FBS does not affect the morphology of the cells with respect to the control, nor with respect to the synthetic serum, showing that it keeps the properties necessary to promote cell proliferation without side effects on cell physiology.

Cell viability was determined by quantifying HEK 293 cells by a trypan blue exclusion assay, which assesses membrane integrity. Figure 7 shows the cumulative cell viability at day 6 of cells cultured under the three supplement conditions (control FBS, white bar; RNA-free FBS, black bar and; synthetic substitute, gray bar). The result shows that the cumulative cell viability at day 6 does not change between the different supplements used, which indicates that the RNA-free FBS described in the present invention does not show any negative effect on cell viability, so it is safe to use.

These viability and proliferation assays were repeated in other cell lines (Figure 8 and Figure 9 respectively). Figure 8 shows the ratio between the proliferation of cell lines grown in medium supplemented with RNA-free FBS and cell lines grown in medium supplemented with serum without treatment (control). Cells were incubated: 1) HEK 293, 2) HeLa, 3) CHO, 4) MCF7 and 5) MEF grown in medium supplemented with untreated FBS and with RNA-free or substantially RNA-free FBS, obtaining the proliferation ratio under both supplement conditions. As in HEK 293 cells, for all cell lines tested, the proliferation ratio between both supplement conditions is close to 1, indicating that there are no differences in supplement capacity between RNA-free FBS and control FBS.

Figure 9 shows the cumulative viability at day 6 of the same cell lines under the two culture conditions, noting that the viability ratio between both supplement conditions is close to 1. This result shows that there are no significant differences in cell viability when using the serum treated with the method of the invention as compared to the control serum.

To verify that the method is effective in the removal of RNA from the serum, determinations of some miRNAs were carried out before and after the application of the method. For this, RNA was extracted from a commercial FBS product with a rich content of miRNAs, in accordance with what was previously described (Figure 1) and subsequently the relative amount of the most represented miRNAs in the serum was identified, according to a bioinformatic analysis of the massive sequencing results.

The probes used for the determination of each of the miRNAs are described in Table 2. As can be seen in Figure 10, all the miRNAs assessed: 1) Bta-miR-143; 2) Bta-miR-181a; 3) Bta-miR-192; 4) Bta-miR-380-3p and 5) Hsa-miR-25-3p were found in the control serum (white bars), while no miRNA was detected below 38 Ct (dotted line) in the serum treated with the method of the invention (black bars), which indicates that the miRNAs in greater amount of the FBS were removed or reduced below the detection limit when applying the method of the invention.

There are commercial FBS products that have low miRNA content but contain significant amounts of other RNAs (Figure 1, bars 4 and 7). Computational analysis of the several classes of RNA contained in one of these commercial products indicated a rich content in two RNA sequences: the RNA classified as U47 and another RNA classified as "not annotated" (designated here as # 49627).

To corroborate the elimination of other types of RNA, a qRT-PCR was performed with specific probes to evaluate the amount of these two RNAs. The probes used for the determination of U47 are described in Table 2.

**Table 2**

| miRNA | Assay identifier |
|---|---|
| bta-mi-R-143 | ID: 006735_mat |
| bta-miR-181a | ID: 005861_mat |
| bta-miR-192 | ID: 006776_mat |
| bta-miR-380-3p | ID: 006377_mat |
| hsa-miR-10b | ID: 002218 |
| hsa-miR-25-3p | ID: 000403 |
| hsa-miR-423-5p | ID: 00234 |
| hsa-miR-486 | ID:001278 |
| U47 | ID: 001223 |

For the unannoted RNA sequence # 49627, SYBR-green type fluorescence was used using the previously reported technique, using the primer oligonucleotides described in Table 3.

**Table 3**

| Primer | Sequence |
|---|---|
| 1 | 5'GTCGTATCCAGTGCAGGGTCCGAGGTATTCGCACTGGATACGACGACGTG-3' (stem and handle) |
| 2 | 5'-CGGAATGTGGAACCACCCA-3' (sense) |
| 3 | 5'-GTCGTATCCAGTGCAGGGT-3' (reverse) |
| 4 | 5'-CUACGGAAUGUGGAACCACCCACGAGGCCACGUC-3' (RNA # 49627) |

Figure 11 shows the amount of 1) U47 and 2) # 49627 in the untreated commercial product (white bars) and in the same serum after being treated with the method of the invention (black bars). Both RNAs were found at levels below 20 Ct in untreated serum, and the application of the method of the invention removes both types of RNAs, reducing the levels thereof below the detection limit of 38 Ct.

An important aspect of those commercial products that seem to be free of miRNAs is that, despite having undetectable levels of some miRNAs such as Bta-miR-143; Bta-miR-181a; Bta-miR-192; Bta-miR-380-3p and Hsa-miR-25-3p, they do contain some miRNAs in still detectable amounts. Figure 12 shows a qRT-PCR analysis for the determination of 1) Hsa-miR-486; 2) Hsa-miR-423-5p; and 3) Hsa-miR-10b in one of these commercial products before treatment (white bars) and after applying the method of the invention (black bars). It is noted that in untreated FBS, these miRNAs were detected around 34 Ct and that when applying the method of the invention these miRNAs decreased below the detection limit of 38 Ct.

In accordance with the description made in the present application, the invention described herein relates to RNA-free or substantially RNA-reduced mammalian sera and to the methods of obtaining said RNA-free or substantially RNA-reduced sera. The sera obtained by the present method maintain their ability to supplement cell cultures and do not contain chemical residues potentially harmful to cell culture.

To put into practice the method of the present invention, any methodology described in the prior art that is useful for the controlled heating of a serum sample can be used, preferably in sterile containers or vessels, or in industrial containers, including but not limited to, bathing and immersion in temperature-controlled water, incubation in temperature-controlled cabinets (furnace) or the use of any other controlled heating device.

Temperatures between 52°C and 63°C, preferably between 55°C and 57°C, can be used for the serum heating process. The heating time can range between 25 and 60 minutes, preferably 35 minutes.

The cooling process can be stepwise after heating, either without manipulating the temperature by allowing it to cool down to room temperature, or by using cooling means to speed up the process, for example, immersion in water or liquids at or below room temperature or using any other controlled cooling device. The final cooling temperature can range between 8°C and 25°C, preferably 16°C.

For the alkalinization process, several alkaline or basic chemical compounds or salts can be used, in solution or anhydrous, which release hydroxyl ions (OH-), including, but not limited to: potassium hydroxide (KOH), magnesium hydroxide (Mg(OH)₂), calcium hydroxide (Ca(OH)₂), sodium hydroxide (NaOH), and others of a similar nature. One skilled in the art can routinely standardize optimal temperatures and times for the heating and cooling processes required in the present method.

The alkali concentrations can range between 10-12 N, and preferably 12 N. One skilled in the art can routinely standardize the optimal concentrations for the alkalinization process required in the present method.

The serum should be alkalized to a pH between 10 and 12, preferably a pH of 12.

The exposure time of serum to alkali can be varied according to the RNA concentration present and the volume, but it must be kept for a minimum of 3 minutes, and can range between 3 and 20 minutes, preferably between 5 and 10 minutes.

For the neutralization process, several compounds or salts of an acidic chemical nature can be used, including, but not limited to: phosphoric acid (H₃PO₄), nitric acid (HNO₃), acetic acid (CH₃COOH), hydrochloric acid (HCI), among others of a similar nature.

The acid concentrations can range between 0.1 and 2 N, and preferably 1 N can be used as the concentration. One skilled in the art can routinely standardize the optimal concentrations for the neutralization process required in the present method.

The serum must be acidified until obtaining a physiological pH between 7.2 and 7.5, preferably pH 7.4.

In a preferred embodiment of the invention, the serum is heated at a temperature between 55°C and 57°C for 30 to 45 minutes; it is allowed to cool gradually until reaching 20°C room temperature, the serum is alkalized using anhydrous NaOH (powder or granular), until reaching pH 12 for 10 minutes and subsequently the pH of the alkalized serum is reduced using HCI in 1N concentration, until achieving a neutralization at a physiological pH of 7.4.

In case the DNA concentration is very high, for example, above 45 or 50 ng/mL, the method of the invention can be applied in consecutive iterations to ensure the removal of RNA.

In one embodiment of the invention, the method described in the present application can integrate an additional ultracentrifugation step between 80,000 xg and 100,000 xg. If ultracentrifugation is carried out, and the process jeopardizes the sterility of the serum, an additional sterilization step must be carried out using any of the techniques known in the prior art, for example filtration through 0.2-micron pore membranes.

Any type of animal serum can be used to practice the present invention, including, but not limited to, bovine, equine, human, mouse, rat and goat serum, in addition to the respective fetal serum variants.

This invention is further illustrated by the following examples, which are in no way construed as limitations imposed on the scope of the claims. On the contrary, these examples are presented for a better understanding of the practice of the invention, with the understanding that they only represent some of the embodiments of the invention.

### DESCRIPTION OF A WAY TO CARRY OUT THE INVENTION

### EXAMPLE 1

### Removal of RNA from FBS.

Starting from a regular FBS commercial product, an initial determination of the total RNA concentration was carried out by means of the extraction thereof with the Trizol product following the manufacturer's recommendations, finding a concentration of 35 ng per mL.

The FBS was heated at 56°C for 35 minutes, slowly allowed to cool to room temperature until reaching 20°C. Subsequently, the serum was alkalized using 12 N anhydrous NaOH, until reaching pH 12 and it was maintained that way for 15 minutes and later the pH of the alkalized serum was reduced using 1N HCl, until reaching a physiological pH of 7.4.

After applying the method, the total RNA concentration was determined by the Trizol extraction method mentioned above, finding a concentration below the detection limit by spectro-fluorometry.

### EXAMPLE 2

### Removal of RNA from FBS combining the method of the invention and ultracentrifugation.

Starting from a regular FBS commercial product, whose RNA concentration was found to be 40 ng/mL, the serum was subjected to ultracentrifugation of 100,000 xg for 7 hours at 4°C. At the end of the ultracentrifugation, the sample supernatant was transferred to a new, sterile container without disturbing the resulting button. To preserve sterile conditions, the serum supernatant was passed through a sterile filter with a 0.2 microns pore size.

Subsequently, the FBS supernatant was subjected to heating at 56°C for 35 minutes, it was allowed to slowly cool to room temperature until reaching 20°C; Subsequently, the serum was alkalized using anhydrous NaOH, until reaching pH 12 and it was maintained that way for 15 minutes, later, the pH of the alkalinized serum was reduced using 1N HCI, until reaching a physiological pH of 7.4.

After applying the method, the total RNA concentration was determined by the Trizol extraction method mentioned above, finding a concentration below the detection limit by spectro-fluorometry.

### EXAMPLE 3

### Removal of RNA from FBS using the method of the invention in different iterations

Different serum packaging are expected to contain higher amounts of RNA than others. In case of having serum samples with high RNA content, the method of the invention can be iterated to yield an additive result of its efficiency in the RNA removal.

Starting from a serum whose RNA content was quantified above 45 ng/mL, it was subjected to heating at 56°C for 35 minutes, it was allowed to slowly cool to room temperature until reaching 20°C; subsequently, the serum was alkalized using anhydrous NaOH, until reaching pH 12 and it was maintained that way for 15 minutes, later, the pH of the alkalinized serum was reduced using 1N HCI, until reaching a physiological pH of 7.4.

Subsequently, the heating, cooling, alkalinization and neutralization processes were repeated under the same conditions.

After applying the method in two consecutive iterations, the total RNA concentration was determined by the Trizol extraction method mentioned above, finding a concentration below the detection limit by spectro-fluorometry.

## Claims

1. An animal serum **characterized in that** it is RNA-free or substantially RNA-free.

2. The serum according to claim 1, wherein the serum is of bovine, equine, human, mouse, rat or goat origin, or one of the variants of the respective fetal sera.

3. The serum according to any of claims 1 to 2, wherein that the serum is fetal bovine serum.

4. A useful method to remove RNA from mammalian serum, said method comprsing: a) heating the serum to a temperature between 52°C and 63°C, b) cooling the serum to a temperature between 8°C and 25°C, c) alkalizing the serum to a pH between 10 and 12 and, d) neutralizing the serum to a physiological pH, wherein these steps are carried out in this sequential order.

5. The method according to claim 4, wherein the serum is of bovine, equine, human, mouse, rat or goat origin, or one of the variants of the respective fetal sera.

6. The method according to any of claims 4 and 5, wherein the serum is heated to a temperature between 55°C and 57°C.

7. The method according to any of claims 4 to 6, wherein the temperature is maintained for 30 to 45 minutes.

8. The method according to any of claims 4 to 7, wherein the alkalinization of the serum is carried out until reaching pH 12.

9. The method according to any of claims 4 to 8, wherein the alkalinization of the serum is carried out using a compound of a basic chemical nature selected from the group comprising: sodium hydroxide (NaOH), potassium hydroxide (KOH), magnesium hydroxide (Mg(OH)₂), calcium hydroxide (Ca(OH)₂), among others.

10. The method according to any of claims 4 to 9, wherein the alkalinization is maintained for a period of 3 to 20 minutes.

11. The method according to any of claims 4 to 10, wherein the alkalinization is maintained for a period of 5 to 10 minutes.

12. The method according to any of claims 4 to 11, wherein the neutralization of the serum is carried out using a compound of an acidic chemical nature selected from the group comprising: phosphoric acid (H₃PO₄), nitric acid (HNO₃), acetic acid (CH₃COOH), hydrochloric acid (HCI), among others.

13. The method according to any of claims 4 to 12, wherein the neutralization of the serum is carried out until reaching a pH between 7.2 and 7.5.

14. The method according to any of claims 4 to 13, wherein the neutralization of the serum is carried out until reaching pH 7.4.

15. The method according to any of claims 4 to 14, further comprising an initial or final ultracentrifugation step.

16. A method useful to remove RNA from mammalian serum, said method comprising: a) heating of the serum to a temperature between 52°C and 63°C, b) cooling of the serum to a temperature between 8°C and 25°C, c) ultracentrifugation of the serum between 80,000 xg and 100,000 xg for at least 5 to 7 h, wherein these steps are carried out in this sequential order.

17. The method according to claim 16, wherein the serum is of bovine, equine, human, mouse, rat or goat origin, or one of the variants of the respective fetal sera.

18. The method according to any of claims 16 to 17, wherein the serum is heated to a temperature between 52°C and 63°C.

19. The method according to any of claims 16 to 18, wherein the temperature is maintained for 30 to 45 minutes.

20. The method according to any of claims 16 to 19, wherein the ultracentrifugation is carried out at 100,000 xg for 7 hours.

21. The method according to any of claims 16 to 20, wherein the ultracentrifugation is carried out for 5 to 10 h.

22. The method according to any of claims 16 to 18, further comprising an additional serum sterilization step.
